# EUROPEAN PATENT APPLICATION

(11) **EP 1 835 428 A2**
(43) Date of publication of application: **19.09.2007**
(21) Application number: 07250747.8
(22) Date of filing: 22.02.2007
(51) Int. Cl.: G06F 19/00

(54) **An application server for processing medical image data**

(30) Priority: 09.03.2006 US 780601 P; 01.05.2006 US 414958
(71) Applicant: iCad, Inc., Beavercreek, Ohio 45431 (US)
(72) Inventor: Woods, Kevin S., Beavercreek, Ohio 45431 (US); Collins, Michael J., Beavercreek, Ohio 45431 (US); Mc Ginnis, Ryan Edward, Tipp City, Ohio 45371 (US)
(74) Representative: Cummings, Sean Patrick

(57) **Abstract**

A method and system for sharing intermediate medical image processing results among several different applications is disclosed. An application server serves as mediator between a medical image database storage, image processing applications, and a workstation display resident on a computer network. The application server polices the intermediate processing results from several different image processing applications in order for the image processing applications to share their intermediate results. By sharing the intermediate results, the application server helps to eliminate duplicate processing and inconsistent results between the image processing applications. The final image processing results are either stored on the medical image database or displayed on the workstation as needed. The image processing applications could reside on the application server or on their own separate networked computer. Alternatively, some image processing applications could reside on the application server and some on separate networked computers.

## Description

### CROSS-REFERENCE TO RELATED APPLICATIONS

This application claims the benefit of U.S. Provisional Application Serial No. 60/780,601 (ICA 0104 MA), filed March 9, 2006.

### BACKGROUND OF THE INVENTION

The present invention generally relates to a method and system for processing medical image data and, in particular, relates to a method and system for processing medical image data in which different applications share intermediate processing results.

Many different applications for processing medical image data exist and are used today. For example, there is visualization software in which the medical image is presented for review by a medical professional on a workstation display, or computer monitor. In addition, there is computer-aided detection (CAD) software in which abnormalities are automatically detected in the medical image data by computer and these CAD results are presented to the medical professional. Many times these image processing applications come from different vendors. However, many of the same steps taken while processing the medical image data are often required by the different image processing applications.

One such area of medical image processing is virtual colonography (VC). VC uses x rays and computers to produce two- and three-dimensional images of the colon (large intestine) from the lowest part, the rectum, all the way to the lower end of the small intestine and display them on a screen. The procedure is used to diagnose colon and bowel disease, including polyps, diverticulosis, and cancer. VC can be performed with computed tomography (CT).

Using VC as an example, the visualization software will process the three-dimensional image data and render a virtual view of the large bowel for presentation and review by a medical professional. In order to render the large bowel, certain preprocessing steps are required. The anatomical region of interest, the colon in this case, must be segmented from the rest of the image data. It may also be necessary to electronically remove fluid and/or stool from the colon region. Next, the visualization software can take the segmented, electronically cleansed colon image data and render a three-dimensional model for display. At the same time, a CAD application is used to detect polyps in the colon. The CAD application must first segment the colon and electronically cleanse it in the same way the visualization application was required to do. Once this has been done, a detection algorithm will be used to find suspicious polyps in the colon image data.

In this example, both the visualization application and the CAD application were required to perform identical processing steps. Since each of these image processing applications typically comes from a different software vendor, the same steps are repeated for each image processing application. In addition, each vendor will have developed their own image processing methods leading to different results for the same step. That is, the segmented colon produced by the visualization vendor's software will not be the same as the segmented colon produced by the CAD vendor's software. This can lead to inconsistency problems when displaying both a rendered colon and CAD marks on the same workstation. For example, a CAD mark may be generated on a region that does not lie within the rendered colon due to different image processing methods being used by the different application vendors. Therefore, it takes longer for the correct resulting image to be resolved and rendered by the different vendors.

If the image processing applications are broken down into smaller separate image processing applications which save intermediate results that can then be used by the other image processing applications, it becomes possible to avoid duplicating many of the image processing tasks. For example, a separate image processing application to segment the anatomical region of interest, in this example the colon, would process the image data first and save the results. A separate electronic cleansing image processing application would further process the output of the colon segmentation application and remove fluid and stool. Again, the intermediate results would be saved. Now, the rendering application and the CAD application could each process the segmented cleansed colon data separately.

Therefore, there is a need for an application server to facilitate an integrated visual image solution when complex image processing applications come from multiple vendors are to be displayed on a workstation.

There is also a need for an application server to facilitate an integrated visual image solution when there is only a single vendor providing multiple image processing applications are to be displayed on a workstation.

### BRIEF SUMMARY OF THE INVENTION

According to the present invention, a method and system for sharing intermediate medical image processing results among several different applications is disclosed. An application server serves as mediator between a medical image database storage, image processing applications, and a workstation display resident on a computer network. The application server polices the intermediate processing results from several different image processing applications in order for the image processing applications to share their intermediate results with each other. The final image processing results are either stored on the medical image database or displayed on the workstation as needed. The image processing applications could reside on the application server or on their own separate networked computer. Alternatively, some image processing applications could reside on the application server and some on separate networked computers.

In accordance with one embodiment of the present invention, the application server reduces duplicate processing by the several different image processing applications by coordinating the sharing of intermediate image processing results among the different image processing applications.

Accordingly, it is a feature of the embodiments of the present invention to significantly reduce the computing time required for all image processing applications to completely process the medical image data and produce final results by eliminating duplicate processing by the image processing applications.

It is another feature of the embodiments of the present invention to avoid inconsistencies in the final results by performing all subsequent processing on identical versions of the preprocessed image data.

Other features of the embodiments of the present invention will be apparent in light of the description of the invention embodied herein.

### BRIEF DESCRIPTION OF THE SEVERAL VIEWS OF THE DRAWINGS

The following detailed description of specific embodiments of the present invention can be best understood when read in conjunction with the following drawings, where like structure is indicated with like reference numerals and in which:

Fig. 1 illustrates a block diagram of the system according to an embodiment of the present invention.

Fig. 2 illustrates another block diagram of the system according to an embodiment of the present invention.

Fig. 3 illustrates yet another block diagram of the system according to an embodiment of the present invention.

### DETAILED DESCRIPTION

In the following detailed description of the embodiments, reference is made to the accompanying drawings that form a part hereof, and in which are shown by way of illustration, and not by way of limitation, specific embodiments in which the invention may be practiced. It is to be understood that other embodiments may be utilized and that logical, mechanical and electrical changes may be made without departing from the spirit and scope of the present invention.

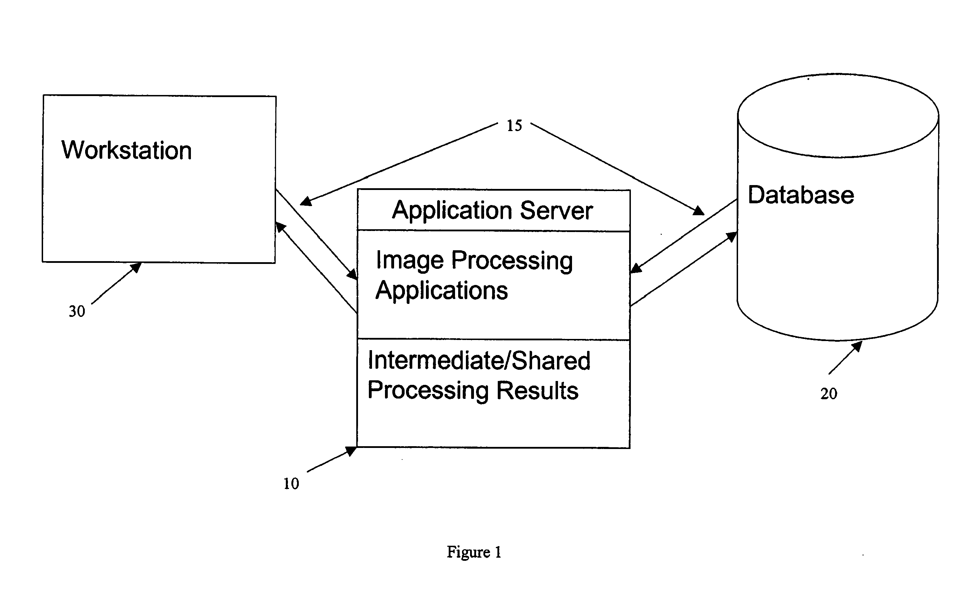

Referring initially to Fig. 1, the original medical image data to be used by the different image processing applications 12 may be stored in a medical image storage database 20. Typically, the type of database used for medical imagery can be a Picture Archiving and Communications System (PACS) and the medical image data can be stored in Digital Imaging and Communications in Medicine (DICOM) format. However, other types of medical image storage databases may be used as well as well other types of format for data storage. The medical image storage database 20 can be connected to a computer network 15. The medical image data can be displayed and reviewed on a workstation 30 which may also be connected to the computer network 15. An application server 10 can serve as a liaison between the medical image storage database 20 and the workstation 30. The application server 10 may also monitor and mediate the use of the medical image data by the different image processing applications 12. The different image processing applications 12 may include, for example, visualization applications, computer-aided detection (CAD) applications, medical image rendering applications, anatomical regions of interest segmentation applications, medical image cleansing applications, or any other type of medical image processing application.

The application server 10 could be implemented in several ways. In one embodiment as illustrated in Fig. 1, the different image processing applications 12 could all exist on a single application server 10 which resides on a computer network 15 along with the medical image storage database 20 and workstation 30. Intermediate processing results 14 would be produced by image processing applications 12 common to all the different image processing applications 12 such as, for example, visualization, anatomical regions of interest segmentation, and cleansing applications. These intermediate processing results 14 then would be stored locally on the application server 10 and every image processing application 12 such as, for example, the rendering and CAD applications, could then be able to readily access the intermediate processing results 14. Event coordination and tracking protocols of the image data sets would be handled by the application server 10 for coordination of the image data sets between the image processing applications 12. For example, in the case of VC, the CAD application could not proceed on a particular set of medical image data until the colon segmentation application finished its processing and made its results available to the application server 10.

Final image processing results after all the image processing applications 12 have finished their processing could then be sent back to the medical image storage database 20 for storage, in DICOM format for example. Alternatively, the final imaging processing results could be called up on the workstation 30 directly from the application server 10 for review by a user, such as, for example, a radiologist, as needed by the user. The status of the processing of the medical image data set would be tracked for coordination. In one embodiment, the status of the image data would be tracked on a table resident on the application server 10.

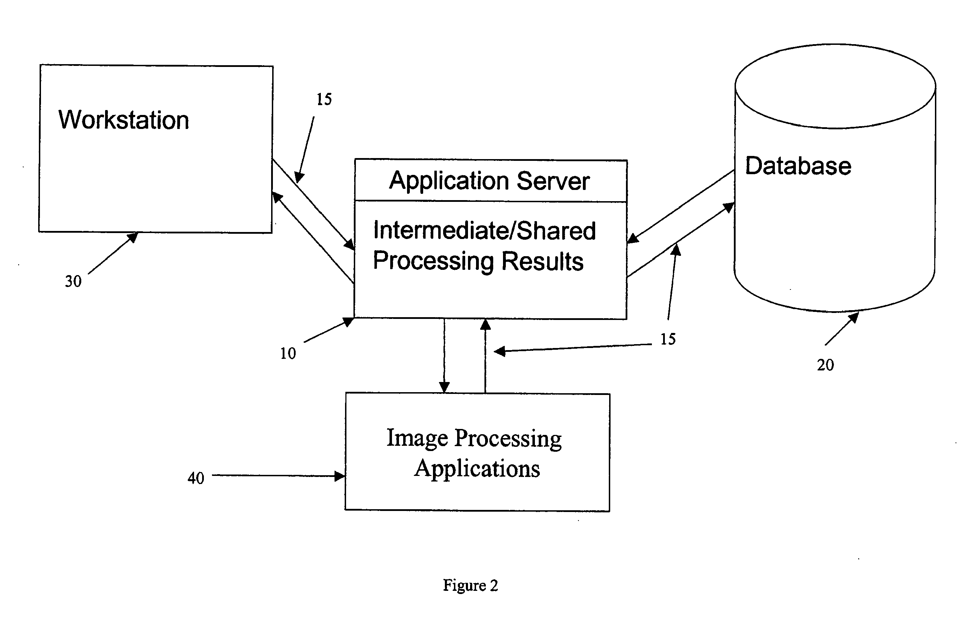

In another embodiment, as illustrated in Fig. 2, each image processing application 13 could reside on its own dedicated networked computer 40 on the computer network 15 and there would simply be a central image storage location on the application server 10, for example, for the intermediate image processing results 14 from the different separately networked image processing applications 13. Again, event coordination and tracking protocol of the medical image data sets could be handled by the application server 10 for coordination and monitoring of the medical image data sets between the different image processing applications 13.

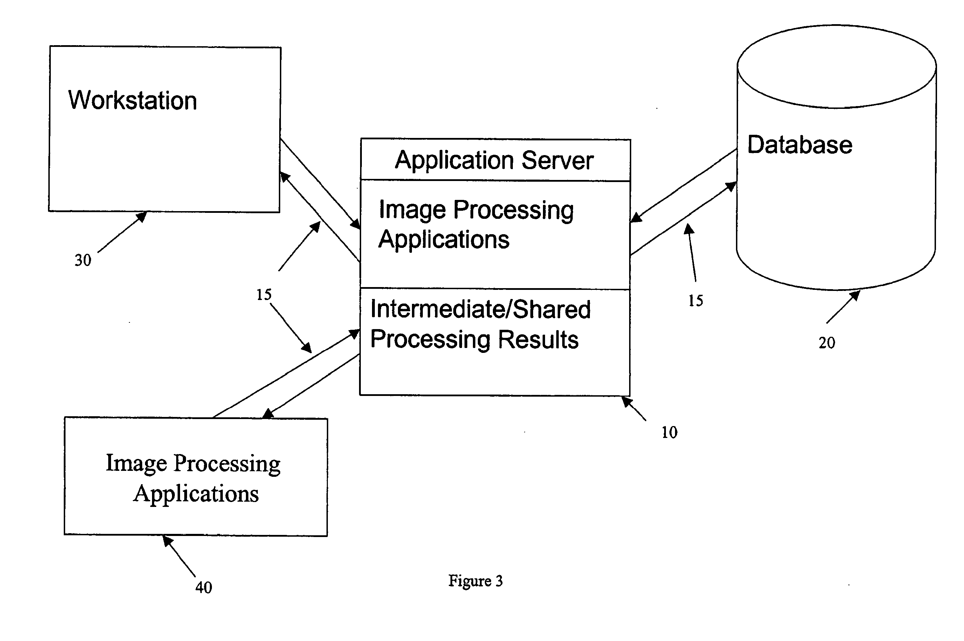

It is also possible that some of the image processing applications 12 would not require a dedicated standalone computer on the computer network while other image processing applications 13 would required a dedicated networked standalone computer. In this embodiment, as illustrated in Fig. 3, some of the image processing applications 12 could reside on the application server 10 while some of the other image processing applications 13 would exist on their own dedicated computer 40 somewhere on the computer network 15. The sharing of intermediate processing results 14 between the different image processing applications 12, 13 would be monitored and controlled by the application server 10 through event coordination and tracking protocols resident on the application server 10.

Therefore, in one embodiment, the different image processing applications 12 as well as the image results, intermediate 14 or otherwise, can reside on a single network computer, the application server 10, in order to facilitate sharing of the intermediate image processing results 14. In another embodiment, the image processing applications 13 may also exist on separate network computers and the application server 10 simply provides a means for sharing image results 14. In yet another embodiment, the image processing applications 12,13 may also exist on separate network computers as well as on the application server 10. In this embodiment, the application server 10 also monitors and controls the sharing of the intermediate image results 14. In all of the cases, the application server 10 assists to eliminate duplicate image processing by the different image processing applications and inconsistent image processing results when different image processing applications are processing the same image data.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

Having described the invention in detail and by reference to specific embodiments thereof, it will be apparent that modifications and variations are possible without departing from the scope of the invention defined in the appended claims. More specifically, although some aspects of the present invention are identified herein as preferred or particularly advantageous, it is contemplated that the present invention is not necessarily limited to these preferred aspects of the invention.

## Claims

1. A system for sharing intermediate medical image processing results among several different image processing applications, the system comprising:
a computer network;
image processing applications for processing medical images, wherein the image processing applications are accessible by the network;
a medical image storage database for storing the medical images used by the image processing applications and for storing the final medical image results of the image processing applications, wherein the medical image storage database is connected to the network;
a workstation for displaying the final medical image results of the image processing applications for review by a user, wherein the workstation is connected to the network; and
an application server for mediating the sharing of the intermediate results of the image processing applications between the image processing applications, wherein the application server is connected to the network, the medical image storage database and the workstation display.

2. The system of claim 1, wherein the medical image storage database is a PACS.

3. The system of claim 1, wherein the medical images are stored in DICOM format on the medical image storage database.

4. The system of claim 1, wherein the image processing applications reside on the application server.

5. The system of claim 1, wherein the image processing applications reside on a computer connected to the network which is communicable connected to the application server.

6. The system of claim 1, wherein the image processing applications reside on both the application server and a computer connected to the network which is communicable connected to the application server.

7. The system of claim 1, wherein the image processing applications comprise visualization applications, rendering applications, computer-aided detection applications, anatomical regions of interest segmentation applications, image cleansing applications, or combinations thereof.

8. The system of claim 1, wherein the intermediate medical image processing results reside on the application server

9. The system of claim 1, wherein the image processing applications and the intermediate medical image processing results reside on the application server

10. A method for sharing intermediate medical image processing results among several different image processing applications, the method comprising:
storing the intermediate medical image processing results on an application server;
accessing the intermediate medical image processing results by the different image processing applications;
monitoring the accessing of the intermediate medical image processing results by an application server;
tracking the status of the image data by the application server; and
storing final image processing results on a medical image storage database.

11. The method of claim 10, wherein the monitoring the accessing of the intermediate medical image processing results by an application server is performed by event coordination.

12. The method of claim 10, wherein the monitoring the accessing of the intermediate medical image processing results by an application server is performed by tracking protocols.

13. The method of claim 10, wherein the tracking of the status of the image data is through the use of a table stored on the application server.

14. The method of claim 10, wherein the storing the final image processing results is stored in DICOM format on a PACS.

15. The method of claim 10, further comprising:
segmenting anatomical regions of interest from medical images by an image processing application; and
storing the segmented anatomical regions of interest as intermediate medical image processing results for use by the several different image processing applications.

16. The method of claim 10, further comprising:
connecting the application server to the medical image storage database by a network.

17. The method of claim 10, further comprising:
connecting the application server to the medical image storage database and to the several different image processing applications through a network.

18. The method of claim 10, further comprising:
displaying the final image processing results on a workstation for review by a user.

19. The method of claim 18, further comprising:
connecting the application server to the medical image storage database and the workstation through a network.

20. The method of claim 18, further comprising:
connecting the application server to the medical image storage database, to the workstation and to the several different image processing applications through a network.

21. A method for sharing intermediate medical image processing results among several different image processing applications, the method comprising:
segmenting out anatomical regions of interest from medical images;
storing the segmented anatomical regions of interest as intermediate medical image processing results;
monitoring the sharing of the intermediate medical image processing results by an application server for use by the several different image processing applications;
tracking the status of use by the intermediate medical image processing results by the application server;
storing the final results of the medical image processing by the several different image processing applications on a medical image storage database, wherein the medical image storage database is connected to the application server by a network; and
displaying final image processing results on a workstation, wherein the workstation is connected to the application server by the network.

22. The method of claim 21, wherein storing the intermediate medical image processing results is on the application server.

23. The method of claim 21, wherein monitoring of the sharing of the intermediate medical image processing results by an application server for use by the several different image processing applications is over the network.
